(19) Europäisches Patentamt
European Patent Office
Office européen des brevets

(11) Publication number: **0 554 744 A1**

(12) **EUROPEAN PATENT APPLICATION**

(21) Application number: **93101069.8**

(51) Int. Cl.5: **C07C 323/47**, A01N 35/10

(22) Date of filing: **25.01.93**

(30) Priority: **05.02.92 JP 47697/92**

(43) Date of publication of application: **11.08.93 Bulletin 93/32**

(84) Designated Contracting States: **BE CH DE FR GB IT LI NL**

(71) Applicant: **NIHON BAYER AGROCHEM K.K.**
**7-1, Nihonbashi Honcho 2-chome Chuo-ku**
**Tokyo 103(JP)**

(72) Inventor: **Goto, Toshio**
**214-18, Koganei, Kokubunji-machi**
**Shimotsuga-gun, Tochigi(JP)**
Inventor: **Hayakawa, Hidenori**
**3-17-20, Ekiminami-machi**
**Oyama-shi, Tochigi(JP)**
Inventor: **Watanabe, Tamie**
**947-2, Mukoishige, Ishige-machi**
**Yuki-shi, Ibaragi(JP)**
Inventor: **Watanabe, Yukiyoshi**
**3-8-6, Higashi**
**Hasuda-shi, Saitama(JP)**
Inventor: **Yanagi, Akihiko**
**1-21-1, Ekiminami-machi**
**Oyama-shi, Tochigi(JP)**

(74) Representative: **Schumacher, Günter, Dr. et al**
**c/o Bayer AG Konzernverwaltung RP**
**Patentabteilung**
**W-5090 Leverkusen 1 Bayerwerk (DE)**

(54) **Cyclohexane diones.**

(57) The invention relates to novel cyclohexane diones of the general formula (I)

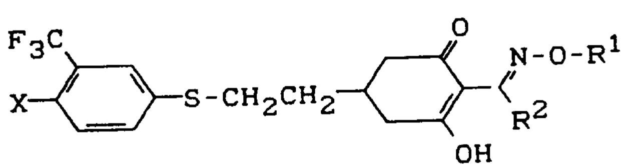

(I)

wherein

$R_1$ represents $C_{1-4}$ alkyl, $C_{2-4}$ alkenyl, $C_{3-5}$ alkynyl or $C_{2-4}$ haloalkenyl,

$R_2$ represents $C_{1-4}$ alkyl, and

X represents halogen,

to a process for their preparation and to their use as herbicides.

EP 0 554 744 A1

The present invention relates to novel cyclohexane diones to a process for their preparation, and to their use as herbicides.

It has already been disclosed that a certain group of cyclohexane derivatives is useful as herbicides (see Japanese Laid-Open Patent Application No Hei 1-13066, U.S. Patent Nos. 462276 and 4741768).

There have now been found novel cyclohexane diones of the general formula (I)

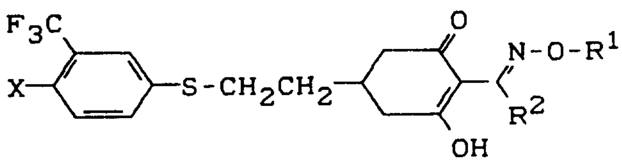

wherein

$R^1$ represents $C_{1-4}$ alkyl, $C_{2-4}$ alkenyl, $C_{3-5}$ alkynyl or $C_{2-4}$ haloalkenyl,

$R^2$ represents $C_{1-4}$ alkyl, and

X represents halogen.

Cyclohexane diones of the formula (I) are obtained when

a) compounds of the formula (II)

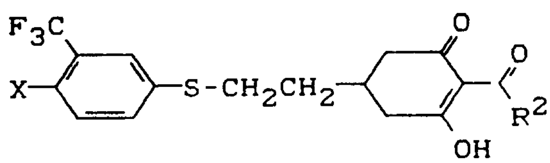

wherein $R^2$ and X have the above mentioned meanings, are reacted with compounds of the formula (III)

$H_2NOR^1$ (III)

wherein $R^1$ has the above mentioned meanings,

if appropriate, in the presence of acid binders, and, if appropriate, in the presence of inert solvents,

The novel cyclohexane diones of the formula (I) exhibit powerful herbicidal properties.

Suprisingly, the cyclohexane diones according to the invention exhibit a substantially stronger selective herbicidal action, than those known from the prior art, for instance, the aforementioned Japanese Laid-Open Patent Application No. Hei 1-13066, and U.S. Patent Nos. 4626276 and 4741768.

In the compounds represented by the formula (I) according to the present invention as well as in each of the general formulas representing the intermediate compounds for the production of the compounds of the present invention, halogen includes fluorine, chlorine, bromine, and iodine, preferably chlorine or fluorine, and $C_{1-4}$ alkyl group includes methyl, ethyl, n-propyl, isopropyl, n-butyl, iso-butyl, sec-butyl, tert-butyl, preferably methyl, ethyl, n-propyl and isopropyl, and more preferably methyl, ethyl, and n-propyl.

$C_{2-4}$ alkenyl group includes allyl, 1-propenyl, 1-butenyl and 2-butenyl, each substituted with fluorine, chlorine, bromine or iodine, and preferably allyl, which is optionally substituted by chlorine.

$C_{3-5}$ alkynyl group includes propargyl, 1-butenyl, 2-butynyl, 1-pentynyl, 2-pentynyl, 3-pentynyl, and 4-pentynyl, and preferably propargyl.

Among the cyclohexane diones according to the invention of the formula (I), the preferred compounds are those in which

$R^1$ represents methyl, ethyl, n-propyl, allyl, propargyl or chlorine-substituted allyl group,

$R^2$ represents methyl, ethyl or n-propyl, and

X represents fluorine or chlorine.

Very particularly preferred cyclohexane diones of the formula (I) are those in which

$R^1$ represents methyl or ethyl,

$R^2$ represents methyl, ethyl, n-propyl and

X represents fluorine or chlorine.

As single dislcosed compounds of the formula (I) according to the invention may be mentioned:

2-(1-ethoxyaminopropylidene)-5-[2-(4-chloro-3-trifluoromethylphenylthio)ethyl]cyclohexane-1,3-dione,

2-(1-ethoxyaminopropylidene)-5-[2-(4-fluoro-3-trifluoromethylphenylthio)ethyl]cyclohexane-1,3-dione,

2-(1-ethoxyaminoethylidene)-5-[2-(4-chloro-3-trifluoromethylphenylthio)ethyl]cyclohexane-1,3-dione,
2-(1-ethoxyaminoethylidene)-5-(2-(4-fluoro-3-trifluoromethylphenylthio)ethyl]cyclohexane-1,3-dione,
2-(1-ethoxyaminobutylidene)-5-[2-(4-chloro-3-trifluoromethylphenylthio)ethyl]cyclohexane-1,3-dione,
2-(1-ethoxyaminobutylidene)-5-[2-(4-fluoro-3-trifluoromethylphenylthio)ethyl]cyclohexane-1,3-dione,
2-(1-(3-chloroallyloxyamino)propylidene)-5-[2-(4-chloro-3-trifluoromethylphenylthio)ethyl]cyclohexane-1,3-dione,
2-(1-(3-chloroallyloxyamino)propylidene)-5-[2-(4-fluoro-3-trifluoromethylphenylthio)ethyl]cyclohexane-1,3-dione,
2-(1-methoxyaminopropylidene)-5-[2-(4-chloro-3-trifluoromethylphenylthio)ethyl]cyclohexane-1,3-dione,
2-(1-methoxyaminopropylidene)-5-[2-(4-fluoro-3-trifluoromethylphenylthio)ethyl]cyclohexane-1,3-dione,
2-(1-propoxyaminoethylidene)-5-[2-(4-chloro-3-trifluoromethylphenylthio)ethyl]cyclohexane-1,3-dione,
2-(1-propoxyaminoethylidene)-5-[2-(4-fluoro-3-trifluoromethylphenylthio)ethyl]cyclohexane-1,3-dione,
2-(1-allyloxyaminopropylidene)-5-[2-(4-fluoro-3-trifluoromethylphenylthio)ethyl]cyclohexane-1,3-dione,
and
2-(1-propargyloxyaminopropylidene)-5-[2-(4-fluoro-3-trifluoromethylphenylthio)ethyl]cyclohexane-1,3-dione.

If, for example, in the process 2-propionyl-5-[2-(4-chloro-3-trifluoromethylphenylthio)ethyl]cyclohexane-1,3-dione and O-ethylhydroxylamine hydrochloride are used as starting materials, the course of the reaction can be represented by the following equation:

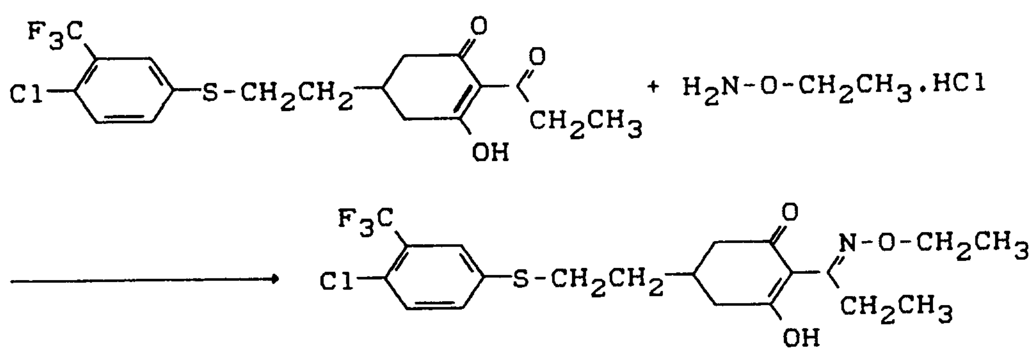

In that process the starting compounds of the formula (II) mean compounds based on the above definitions of $R^2$ and X, preferably substituents based on the above preferred definitions

The compounds of the formula (II) can be obtained by the process disclosed, for example, in Japanese Laid-open Patent Application No. Hei 2-124224. Specific examples of the anilines of the formula (II) are
2-propionyl-5-[2-(4-chloro-3-trifluoromethylphenylthio) ethyl]cyclohexane-1,3-dione,
2-propionyl-5-[2-(4-fluoro-3-trifluoromethylphenylthio)ethyl]cyclohexane-1,3-dione,
2-acetyl-5-[2-(4-chloro-3-trifluoromethylphenylthio)ethyl]cyclohexane-1,3-dione,
2-acetyl-5-[2-(4-fluoro-3-trifluoromethylphenylthio)ethyl]cyclohexane-1,3-dione,
2-butyryl-5-[2-(4-chloro-3-trifluoromethylphenylthio)ethyl]cyclohexane-1,3-dione, and
2-butyryl-5-[2-(4-fluoro-3-trifluoromethylphenylthio)ethyl]cyclohexane-1,3-dione.

Further in the above mentioned process the starting compounds of the formula (III) mean compounds based on the above definition of $R^1$, preferably compounds based on the above preferred definitions.

The compounds of the formula (III) are well known in the field of organic chemistry. As specific examples of the compound (III), there may be mentioned:
O-methylhydroxylamine, O-ethylhydroxylamine, O-allylhydroxylamine, O-(3-chloroallyl)hydroxylamine, O-propargylhydroxylamine and their hydrochlorides.

In carrying out the process as mentioned above, use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, methylene chloride, dichloromethane, chloroform, carbon tetrachloride, 1,2-dichloroethane, chlorobenzene, dichlorobenzene and the like; ethers such as diethyl ether, methyl ethyl ether, di-isopropyl ether, dibutyl ether, dioxane, dimethoxyethane (DME), tetrahydrofurane (THF), diethyleneglycol dimethylether (DGM), and the like; alcohols such as methanol, ethanol, isopropanol, butanol, ethylene glycol; and bases such as pyridine, etc., for example.

The above mentioned process is carried out preferably in the presence of acid binder and as acid binder may be mentioned inorganic bases including hydoxides, carbonates, bicarbonates, and alcoholates of alkalimetals such as, for example, sodium bicarbonate, potassium bicarbonate, sodium carbonate, potassium hydroxide, and calcium hydroxide. As organic bases may be mentioned tertiary amines, dialkylaminoanilines, and pyridines such as, for example, triethylamine, tributylamine, 1,1,4,4-tetramethylenediamine(TMEDA), N,N-dimethylaniline, N,N-diethylaniline, pyridine, 4-dimethylamino pyridine (DMAP), 1,4-diazabicyclo[2,2,2] octane(DABCO), 1,8-diazabicyclo[5,4,0]-undec-7-ene(DBU), sodium acetate, potassium acetate, etc.

In the above mentioned process the reaction temperature can be varied within a substantially wide range. In general, the reaction is carried out at a temperature from about 0 ° C to 100 ° C, preferably at room temperature.

Further, the reaction is carried out under normal pressure, but a higher or reduced pressure may also be used.

In carrying out the above mentioned process the desired compounds of the formula (I) according to the present invention can be obtained by reacting about 1.0 to 2.0 mol amount of the compounds of the formula (III) and 1.0 mol of the compounds of the formula (II), in a diluent such as methanol and in the presence of 1.0 to 2.0 mol amount of acid binder.

The active compounds according to the invention can be used as defoliants, desiccants, agents for destroying broad-leaved plants and, especially, as weedkillers. By weeds, in the broadest sense, there are to be understood all plants which grow in locations where they are undesired. Whether the substance according to the present invention act as total or selective herbicides depends essentially on the amount used.

The active compounds according to the invention can be used, for example, in connection with the following plants:

<u>Dicotyledon weeds of the genera</u>: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Geleopsis, Papaver and Centaurea.

<u>Dicotyledon cultures of the genera</u>: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

<u>Monocotyledon weeds of the genera</u>: Echninochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaeumum, Sphenoclea, Dactyloctenium, Agrotis, Alopecurus and Apera.

<u>Monocotyledon cultures of the genera</u>: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tre plantings. Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the selective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

The formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and

esters, ketones, such as acetone, methyl ethyl ketone, methyl isobutyl ketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules or organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc. The formulations in general contain from 0.1 to 95% by weight of active compound, preferably from 0.5 to 90% by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.01 and 10 kg of active compound per hectare of soil surface, preferably between 0.05 and 1 kg per ha.

The preparation and use of the active compounds according to the invention can be seen from the following examples.

Preparation Examples:

Example 1

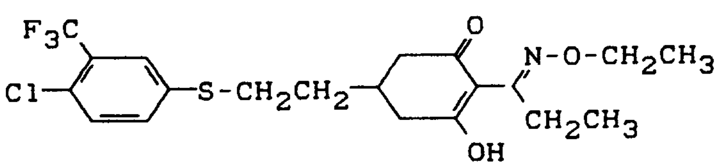

(Compound No. 2)

2-propionyl-5-[2-(4-chloro-3-trifluoromethylphenylthio)ethyl]cyclohexane-1,3-dione (8.53 g), O-ethylhydroxylamine hydrochloride (2.26 g), and sodium acetate (2.06 g) were added in ethanol (100 ml), and the mixture was stirred at room temperature for 24 hours. After evaporation of the solvent, the residue was

made alkaline with saturated sodium carbonate The resulting solution was extracted with ethyl acetate. After removal of the solvent under reduced pressure, the residue was treated through flash column chromatography (hexane: ethyl acetate = 6:1) to obtain 2-(1-ethoxyaminopropylidene)-5-[2-(4-chloro-3-trifluoromethylphenylthio) ethyl]cyclohexane-1,3-dione.
$n_D^{20}$ 1.5553

The compounds obtained according to the present invention with the use of a process similar to the above-mentioned synthesis example are shown as follows:
2-(1-ethoxyaminopropylidene)-5-[2-(4-chloro-3-trifluoromethylphenylthio)ethyl]cyclohexane-1,3-dione (compound No. 1): $n_D^{20}$ 1.5579,
2-(1-ethoxyaminopropylidene)-5-[2-(4-fluoro-3-trifluoromethylphenylthio)ethyl]cyclohexane-1,3-dione (compound No. 3): $n_D^{20}$ 1.5355,
2-(1-ethoxyaminobutylidene)-5-[2-(4-chloro-3-trifluoromethylphenylthio)ethyl]cyclohexane-1,3-dione (compound No. 4): $n_D^{20}$ 1.5537, and
2-(1-(3-chloroallyloxyamino)propylidene)-5-[2-(4-chloro-3-trifluoromethylphenylthio)ethyl]cyclohexane-1,3-dione
(compound No 5): $n_D^{20}$ 1.5604.

Biological test:

Comparative compound:

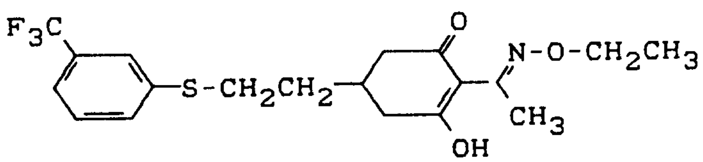

(The compound disclosed in Japanese Laid-Open Patent Application No. Hei 1-13066)

Example 2

Pre-emergence soil treatment test on upland weeds

Formulation of Active Compounds

Carrier: 5 parts by weight of acetone
Emulsifier: 1 part by weight of benzyloxy polyglycol ether

To produce a suitable formulation of each of the active compounds, 1 part by weight of the active compound was mixed with stated amount of carrier and with the stated amount of emulsifier, and the resulting emulsifiable concentrate was diluted with water to the desired concentration.

Test Method

In a greenhouse, a number of pots each having an area of 1000 $cm^2$ were charged with soil obtained from a cultivated field. The seeds of soy bean (Glycine), cotton (Gossypium, fingergrass (Digitaria adscendens), barnyard grass (Echinochloa curs-galli), green foxtail (Setaria viridis), Johnsongrass, and panicum bisulcatum, were sown onto the soil surfaces in the respective test pots, respectively and followed by soil covering thereon. Thereafter, predetermined dosages of the active compound formulation mentioned above were uniformly sprayed onto the soil surfaces of the respective test pots.

Four weeks after the application of the active compound formulations, the degree of herbicidal effects on the weeds and the degree of the phytotoxicity on the crop were determined based on the following assessment rate:

A herbicidal effect of 100% on non-treat soil surface was rated as "completely killed" and the determined degrees of herbicidal effects were rated under the corresponding percentage.

A clearly superior activity compared with the comparison compound, combined with an equally good selectivity in culture plants, is shown in this test for example by the compounds of the preparation examples 1 and 2.

Example 3

Post-emergence foliage treatment on upland field weeds

In a greenhouse, a number of test pots each having an area of 1000 $cm^2$ were charged with soil taken out from a cultivated field, Seeds of soy-bean and cotton were sown onto the soil surface in the respective test pots. Each of the thus sown soil surfaces was covered with a soil layer to a height of 1 cm. Into the soil of the respective soil layers in the respective test pots had beforehand been mixed with the seeds of millet, finger grass, barnyard grass, green foxtail, panicum bisulcatum and Johnsongrass, respectively.

Ten days after the seed sowing and soil-covering when the weeds entered the second leaf stage on average while the soy-beans entered the early leafing stage of true leaves, predetermined dosages of the active compound formulations prepared as in Example mentioned above were uniformly sprayed onto the foliage portions of the test plants in the respective test pots.

Three weeks after the spraying of the active compound formulations, the degree of the herbicidal effect on the degree of the phytotoxicity on the crop were determined in a similar manner as in the above-mentioned Example.

A clearly superior activity compared with the comparison compound, combined with an equally good selectivity in culture plants, is shown in this test for example by the compounds of the preparation examples 1 and 2.

## Claims

**1.** Cyclohexane diones of the general formula (I)

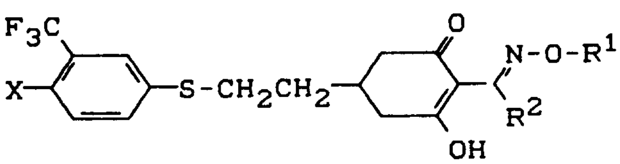
(I)

wherein

- $R^1$ represents $C_{1-4}$ alkyl, $C_{2-4}$ alkenyl, $C_{3-5}$ alkynyl or $C_{2-4}$ haloalkenyl,
- $R^2$ represents $C_{1-4}$ alkyl, and
- X represents halogen.

**2.** Cyclohexane diones of the general formula (I) according to claim 1, wherein

- $R^1$ represents methyl, ethyl, n-propyl, allyl, propargyl or chloro-substituted allyl,
- $R^2$ represents methyl, ethyl or n-propyl and
- X represents fluorine or chlorine.

**3.** Cyclohexane diones of the general formula (I) according to claim 1, wherein

- $R^1$ represents methyl or ethyl,
- $R^2$ represents methyl, ethyl or n-propyl and
- X represents fluorine or chlorine.

**4.** Process for the preparation of cyclohexane diones of the general formula (I)

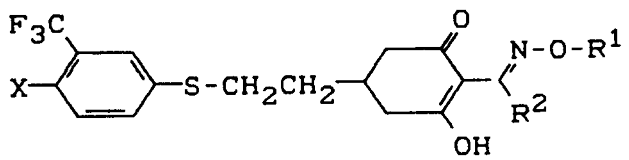

(I)

wherein $R^1$ represents $C_{1-4}$ alkyl, $C_{2-4}$ alkenyl, $C_{3-5}$ alkynyl or $C_{2-4}$ haloalkenyl,
$R^2$ represents $C_{1-4}$ alkyl and
X represents halogen,
characterized in that
compounds of the formula (II)

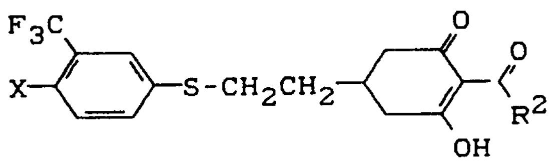

(II)

wherein X and $R^2$ have the above mentioned meanings,
are reacted with compounds of the formula (III)

$H_2NOR^1$ (III)

wherein $R^1$ has the above mentioned meanings, if appropriate, in the presence of acid binders and, if appropriate, in the presence of inert solvents.

**5.** Herbicidal compositions, characterized in that they contain at least one cyclohexane dione of the general formula (I), according to claims 1 to 4.

**6.** Process for combating weeds, characterized in that cyclohexane diones of the general formula (I), according to claims 1 to 4, are allowed to act on weeds and/or their habitat.

**7.** Use of cyclohexane diones of the general formula (I), according to claims 1 to 4, for combating weeds.

**8.** Process for the preparation of herbicidal compositions, characterized in that cyclohexane diones of the general formula (I), according to claims 1 to 4, are mixed with extenders and/or surface active agents.

European Patent Office

# EUROPEAN SEARCH REPORT

Application Number
EP 93101069.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A - 0 457 130 (NIHON BAYER AGROCHEM) * Claims 1,2,4-8 * -- | 1,4-8 | C 07 C 323/47<br>A 01 N 35/10 |
| X | PATENT ABSTRACTS OF JAPAN, unexamined applications, C field, vol. 13, no. 461, October 18, 1989, THE PATENT OFFICE JAPANESE GOVERNMENT page 166 C 645 * No. 1-180 871 (SUMITOMO CHEM CO) * -- | 1,4,5 | |
| D,A | PATENT ABSTRACTS OF JAPAN, unexamined applications, C field, vol. 13, no. 187, May 2, 1989, THE PATENT OFFICE JAPANESE GOVERNMENT page 30 C 592 * No. 1-13 066 (SUMITOMO CHEM CO) * -- | 1,4,5 | TECHNICAL FIELDS SEARCHED (Int. Cl.5) |
| D,A | PATENT ABSTRACTS OF JAPAN, unexamined applications, C field, vol. 13, no. 187, May 2, 1989, THE PATENT OFFICE JAPANESE GOVERNMENT page 30 C 592 * No. 1-13 067 (SUMITOMO CHEM CO.) * -- | 1,4,5 | C 07 C 323/00<br>A 01 N |
| D,A | PATENT ABSTRACTS OF JAPAN, unexamined applications, C field, vol. 13, no. 187, May 2, 1989, THE PATENT OFFICE JAPANESE GOVERNMENT | 1,4,5 | |

The present search report has been drawn up for all claims

| Place of search | Date of completion of the search | Examiner |
|---|---|---|
| VIENNA | 14-04-1993 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-
EP 93101069.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | page 30 C 592 * No. 1-13 068 (SUMITOMO CHEM CO) * -- | | |
| A | EP - A - 0 254 514 (SUMITOMO CHEMICAL) * Abstract * -- | 1,4,8 | |
| A | EP - A - 0 253 537 (SUMITOMO CHEMICAL) * Claims 1,39-41 * -- | 1,4-7 | |
| A | EP - A - 0 082 694 (ICI AUSTRALIA) * Abstract * -- | 1,5 | |
| A | US - A - 4 515 729 (ISAO IWATAKI et al.) * Abstract * -- | 1,5 | |
| A | DE - B - 2 822 304 (NIPPON SODA) * Claim 1 * ---- | 1 | TECHNICAL FIELDS SEARCHED (Int. Cl.5) |

The present search report has been drawn up for all claims

| Place of search | Date of completion of the search | Examiner |
|---|---|---|
| VIENNA | 14-04-1993 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document